# EUROPÄISCHE PATENTSCHRIFT

(11) **EP 2 584 983 B1**
(45) Veröffentlichungstag und Bekanntmachung des Hinweises auf die Patenterteilung: **15.04.2015**
(21) Anmeldenummer: 11730208.3
(22) Anmeldetag: 24.06.2011
(51) Int. Cl.: A61B 17/80, A61B 17/86, A61B 17/00

(54) **KNOCHENPLATTE SOWIE FIXATIONSSYSTEM MIT KNOCHENPLATTE**
BONE PLATE AND FIXATION SYSTEM COMPRISING A BONE PLATE
PLAQUE OSSEUSE ET SYSTÈME DE FIXATION AVEC PLAQUE OSSEUSE

(30) Priorität: 24.06.2010 US 358171 P; 24.06.2010 DE 102010025001
(43) Veröffentlichungstag der Anmeldung: 01.05.2013
(73) Patentinhaber: AAP Implantate AG, 12099 Berlin (DE)
(72) Erfinder: BATSCH, Thomas, 10367 Berlin (DE); PAULIN, Thomas, 14552 Wilhelmshorst OT Michendorf (DE); FISCHER, Hans-Joachim, 12277 Berlin (DE); ALEMU, Bruke, Seyoum, 14195 Berlin (DE)
(74) Vertreter: Blumbach Zinngrebe
(86) Internationale Anmeldenummer: PCT/EP2011/003111
(87) Internationale Veröffentlichungsnummer: WO 2011/160846

(56) Entgegenhaltungen:
- EP-A1- 1 649 819
- EP-A1- 2 016 918
- EP-A2- 1 949 866
- WO-A2-2004/084701
- FR-A1- 2 880 929
- US-A1- 2005 010 226

## Beschreibung

### Gebiet der Erfindung

Die Erfindung bezieht sich auf eine Knochenplatte mit mehreren in Richtung der Plattenlängsachse angeordneten Löchern für die Aufnahme von Knochenschrauben.

### Hintergrund der Erfindung

Bei einer bekannten Knochenplatte dieser Art (EP 0760632 B1) sind.die Löcher zur Oberseite der Knochenplatte hin rundum sphärisch ausgebildet, um eine Knochenschraube mit kugeliger Unterseite des Kopfes in unterschiedlichen Winkelstellungen abstützen zu können. An der Plattenunterseite benachbart zum Knochen weisen die Löcher einen Bereich kleineren Durchmessers mit partiellem Gewinde auf, um auch eine Knochenschraube mit zylindrischem Gewindekopf aufnehmen zu können, die senkrecht zur Plattenebene eingesenkt werden soll.

Bei einer weiteren Knochenplatte der angegebenen Art (EP 1158915 B1 und EP 1158916 B1) sind Langlöcher mit einem Innengewinde, das sich an einem Ende des Langlochs von der Oberseite bis zur Unterseite der Knochenplatte erstreckt und einen Umfangs- oder Zentriwinkel im Bereich von 190-280° aufweist, vorhanden. Das Innengewinde nimmt die gesamte Tiefe des Langloches ein, verjüngt sich gegen die Unterseite der Knochenplatte hin konisch und weist einen Konuswinkel im Bereich von 5 bis 20° auf.

Bei einer weiteren bekannten Knochenplatte (EP 1255498 B1) sind Langlöcher in der Knochenplatte vorgesehen, die oval, ellipsenförmig oder auch rechteckig ausgebildet sein können oder eine Kombination solcher Formen beinhalten; lediglich kreisrunde Löcher sind von dieser Definition des Langlochs explizit ausgeschlossen worden. Das Langloch ist mit einem kreisförmigen Loch kombiniert und dieses ist mit einer dreidimensionalen Strukturierung versehen, welche in Form eines Innengewindes oder einer peripheren Lamelle oder Lippe vorliegt. Dargestellt ist ein konisches Innengewinde, das sich von der Oberseite bis zur Unterseite der Knochenplatte erstreckt und einen Umfangs- oder Zentriwinkel im Bereich von 190 bis 280° aufweist.

Aus DE 19858889 A1 ist ein Fixationssystem für Knochen mit einer Knochenplatte bekannt, welche Langlöcher besitzt, die nahe ihrer dem Knochen benachbarten Unterseite Vorsprünge aufweist, welche sich im unteren Teil des Langloches parallel zur Plattenebene erstrecken. Zur Oberseite der Knochenplatte hin gibt es Sitzflächen für Kugelköpfe der Knochenschrauben. Um mit den Vorsprüngen in der Knochenplatte zusammen zu arbeiten, weist die Knochenschraube unterhalb des Kugelkopfes ein kurzes Stück Gewinde auf, das in der Lage ist, die Vorsprünge in dem Langloch zu deformieren und sich anzuverwandeln. Dabei ist es möglich, die Knochenschraube in verschiedenen Winkelstellungen bezüglich der Achse der Durchgangslöcher einzudrehen.

Knochenplatten, welche mit als Fixationssystem mit Knochenschrauben zusammenwirken, sind ferner aus den Dokumenten WO 2004/0841701 A2, FR 2 880 929 A1, EP 1 949 866 A2; US 2005/010226 A1, EP 1 649 819 A1 und EP 2 016 918 A1 bekannt.

Das Dokument EP 1 859 752 A1, aus dem der Anspruch 1 abgeleitet wird, zeigt eine Knochenplatte, die mit korrespondierenden Knochenschrauben eine Kompression von Knochenfragmenten ermöglicht, wobei die Knochenschrauben eine winkelstabile Verbindung eingeht. Die hierfür vorgesehenen Löcher setzen sich aus einem Langloch und einem angrenzenden Rundloch zusammen, wobei das Rundloch über einen Teil seines Umfangs Gewindezüge aufweist.

### Allgemeine Erfindungsbeschreibung

Der Erfindung liegt die Aufgabe zugrunde, eine Knochenplatte zu schaffen, bei der verschiedenartige Knochenschrauben - solche mit Konussitzflächen und solche mit Kugelsitzflächen - angewendet werden können, um den unterschiedlichen Anforderungen beim Fixieren eines gebrochenen oder geschädigten Knochens zu genügen. Insbesondere sollen Knochenfragmente beim Fixieren relativ zueinander verschoben werden können.

Die Aufgabe der Erfindung wird durch eine Knochenplatte nach Anspruch 1 gelöst. Vorteilhafte Weiterbildungen der Erfindung sind Gegenstand der abhängigen Ansprüche.

Im Einzelnen weist die Knochenplatte einen vorzugweise länglichen Plattenkörper aus gewebeverträglichem, steifen Material auf, der eine Oberseite und Unterseite und eine Längsachse bestimmt. Quer zur Plattenebene sind Lochausbildungen vorgesehen, die aus einem ersten, größeren Rundloch und einem zweiten, kleineren Rundloch bestehen, wobei sich die Rundlöcher unter Bildung von Kanten schneiden, zwischen denen ein Durchlass für den Schraubenschaft einer Knochenschraube gebildet wird. Um beide Rundlöcher herum ist genau eine radiale Rippe vorgesehen, die von der Lochwandung ausgeht und sich in einer Ebene auf die Rundlochmitte hin erstreckt. Es liegt im Rahmen der Erfindung, dass sich diese Rippe in einer Ebene und nicht wie bei einem Gewinde mit einer Steigung versehen erstreckt. Hierdurch kann bei Ausführungsformen mit einer umlaufend geschlossenen beziehungsweise ringförmig geschlossenen Rippe, welches bei einem Gewindegang prinzipiell nicht möglich wäre, eine verbesserte Festigkeit, insbesondere höhere Steifigkeit bereitgestellt werden.

Eine Knochenschraube mit Gewinde am Schraubkopf kann sich an der Rippe des kleineren Rundloches abstützen und einen Eingriff für die Rippe des größeren Rundloches ermöglichen, wodurch gegenseitige Klemmung erfolgt.

Im Sinne der Erfindung kann diese Klemmung auch zu einer elastischen Deformation führen, welche aber nach dem Trennen der Knochenschraube von der Knochenplatte im Wesentlichen keinen bleibenden deformierten Anteil hinterlässt, dies bedeutet, dass diese Deformation nicht über den rein elastischen Anteil hinaus auch noch plastische Deformationen umfasst. Nach dem Trennen der Knochenschraube von der Platte sind folglich keine Grate oder Rillen an der Knochenschraube oder auch an der Knochenplatte zu erkennen. Sollten durch unsachgemäße Benutzung jedoch Oberflächenveränderungen erzeugt werden, liegen diese typischerweise nicht als Rillen, Furchen oder Grate sondern eher als flächige Reibstellen vor.

Gemäß bevorzugter Ausführungsform weist das erste, größere Rundloch drei Abschnitte auf, nämlich einen oberen, rundkehlförmigen Abschnitt oberhalb der umlaufenden Rippe, einen mittleren, rundkehlförmigen Abschnitt unterhalb der umlaufenden Rippe sowie einen unteren, kegelstumpfförmigen Abschnitt, der sich zur Unterseite des Plattenkörpers hin verjüngt und dessen größter Durchmesser kleiner als der Durchmesser des oberen oder mittleren Abschnitts ist. Das kleinere Rundloch umfasst einen oberen Abschnitt mit einem geneigten Übergangsbereich zur Plattenoberseite hin, ferner einen mittleren Abschnitt unterhalb der Ebene der umlaufenden Rippe mit geneigt-abgerundeter Fläche sowie einen unteren, zylindrischen oder konischen Abschnitt mit einem Durchmesser kleiner als der Durchmesser des oberen oder mittleren Abschnitts.

Bei allen Ausführungsformen lassen sich Knochenschrauben mit einem Kopf verwenden, der in seinem oberen Bereich mit Schraubgewinde und in seinem unteren Bereich mit einer konischen Sitzfläche versehen ist, und zwar in der Weise, dass beim Einschrauben senkrecht in einen Knochen eine Verschiebung des Kopfes relativ zu der Lochausbildung stattfindet, dergestalt, dass Knochenbruchstücke beim Fixieren aneinander angenähert werden können. Die neue Knochenplatte ermöglicht aber auch die Anwendung von Knochenschrauben mit sphärischen Sitzflächen an der Unterseite des Schraubkopfes. Solche Kugelkopfschrauben können im Winkel zu der Knochenplatte eingeschraubt werden, wie es mitunter nötig ist.

Weitere Einzelheiten der Erfindung ergeben sich aus der Beschreibung der dargestellten Ausführungsbeispiele und den angefügten Ansprüchen.

### Die Zeichnungen zeigen:

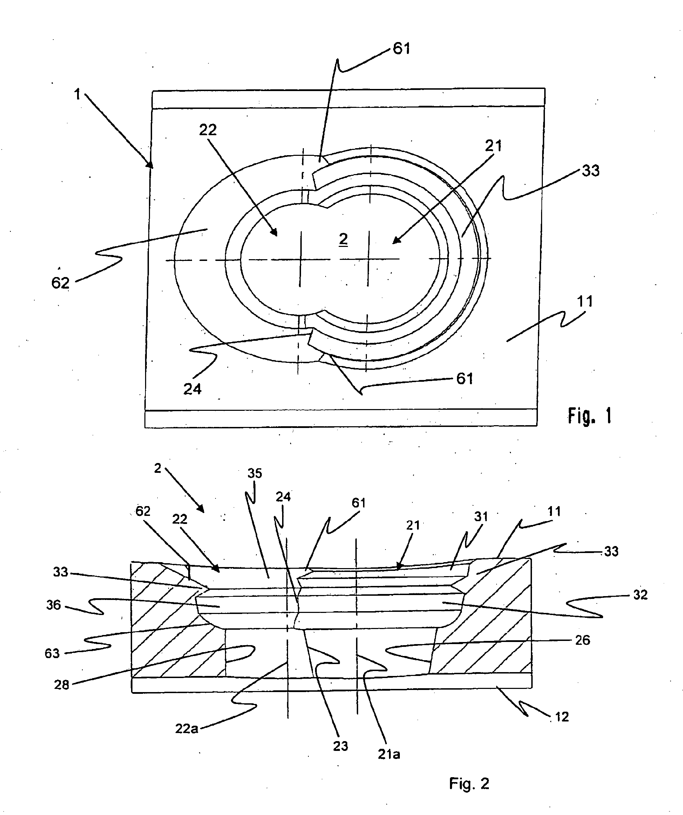

- Figur 1: eine Draufsicht auf eine Lochausbildung in einer Knochenplatte,
- Figur 2: eine Schnittansicht der Lochausbildung,
- Figur 3: eine perspektivische Darstellung der Lochausbildung, und
- Figur 4: eine Knochenschraube im Eingriff mit einer Lochausbildung,
- Figur 5: eine zweite Ausführungsform einer Knochenplatte in perspektivischer Darstellung,
- Figur 6: eine Schnittansicht der Lochaüsbildung,
- Figur 7: eine Seitenansicht der Lochausbildung,
- Figur 8: eine Knochenschraube im Eingriff mit einer Lochausbildung, zusammen mit einer Ausschnittsvergrößerung,
- Figur 9: eine weitere Ausführungsform einer Knochenplatte mit einer umlaufenden Rippe mit verschiedener Höhe in radialer Richtung,
- Figur 10: eine Ausschnittvergrößerung eines Teils einer Querschnittdarstellung entlang der Linie AA und somit parallel zu der Längsachse der in Figur 9 dargestellten weiteren Ausführungsform der Knochenplatte verlaufend mit eingefügter Knochenschraube,
- Figur 11: eine Querschnittdarstellung entlang der Linie AA und somit parallel zur Längsachse der in Figur 9 dargestellten weiteren Ausführungsform der Knochenplatte verlaufend mit eingefügter Knochenschraube,
- Figur 12: eine Ausschnittvergrößerung eines Teils einer Querschnittdarstellung entlang der Linie BB und somit quer zu der Längsachse der in Figur 9 dargestellten weiteren Ausführungsform der Knochenplatte verlaufend mit eingefügter Knochenschraube,
- Figur 13: eine Querschnittdarstellung entlang der Linie BB und somit quer zu der Längsachse der in Figur 9 dargestellten weiteren Ausführungsform der Knochenplatte verlaufend mit eingefügter Knochenschraube,
- Figur 14: eine nochmals weitere Ausführungsform einer Knochenplatte mit einer umlaufenden Rippe mit verschiedener Höhe in radialer Richtung, bei welcher die Symmetrieachse der umlaufenden Rippe relativ zur Symmetrieachse des unter der Rippe liegenden konischen Abschnitts des größeren Rundlochs versetzt angeordnet ist und bei welcher entsprechende Radien um die jeweilige Symmetrieachse strichpunktartig eingezeichnet sind, um deren relativen Versatz einfacher erkennbar zu machen,
- Figur 15: die in Figur 14 dargestellte nochmals weitere Ausführungsform der Knochenplatte mit umlaufender Rippe mit verschiedener Höhe in radialer Richtung jedoch ohne die entsprechenden Radien um die jeweilige Symmetrieachse, um deren tatsächliche Form einfacher erkennbar zu machen,
- Figur 16: eine Querschnittdarstellung der in Figur 14 und 15 dargestellten nochmals weiteren Ausführungsform entlang einer Linie, welche der Linie AA in Figur 9 entsprechen würde und somit parallel zur Längsachse der in Figur 14 und 15 dargestellten nochmals weiteren Ausführungsform der Knochenplatte verlaufend jedoch ohne eingefügte Knochenschraube,
- Figur 17: eine Querschnittdarstellung der in Figur 14 und 15 dargestellten nochmals weiteren Ausführungsform entlang einer Linie, welche der Linie BB in Figur 9 entsprechen würde und somit quer zur Längsachse der in Figur 14 und 15 dargestellten nochmals weiteren Ausführungsform der Knochenplatte verlaufend jedoch ohne eingefügte Knochenschraube.

### Detaillierte Beschreibung bevorzugter Ausführungsformen

Bei der nachfolgenden detaillierten Beschreibung bevorzugter Ausführungsformen bezeichnen um der Klarheit willen gleiche Bezugszeichen im Wesentlichen gleiche Teile in oder an diesen Ausführungsformen.

Die Figuren zeigen das Stück einer Knochenplatte, die aus einem vorzugsweise länglichen Plattenkörper 1 aus gewebeverträglichem, steifem Material besteht und in dem eine Reihe von Lochausbildungen angebracht sind, von denen eine Lochausbildung 2 dargestellt ist. Der Plattenkörper 1 kann außer länglich auch oval, rund oder mehreckig ausgebildet oder von seiner Form her der jeweiligen Anwendung angepasst sein.

Als gewebeverträgliches Material werden beispielsweise Metalle und deren Legierungen verstanden, wie diese üblicherweise zur Herstellung von Implantaten verwendet werden. Bevorzugte Metalle umfassen Titan in jeder Form, vorzugsweise auch dessen Legierungen TiAl6V4 und TiCp. Auch Stähle, wie beispielsweise Implantatestahl, beispielsweise die Legierung 1.4441 sind vorteilhaft verwendbar.

Eine weitere Materialklasse umfasst hierfür auch resorbierbare Materialien, wie Magnesium oder resorbierbare Kunststoffe wie PLA. PLA ist ein biokompatibler und resorbierbarer Kunststoff aus chemisch aneinander gebundenen Milchsäuremolekülen, der wie auch andere resorbierbare Kunststoffe verwendet werden kann.

Als steif wird eine Knochenplatte dann angesehen, wenn diese konstruktiv die für ihre vorgesehene Verwendung und Einsatzzweck nötige Steifigkeit zur Verfügung stellt. Dies kann je nach verwendetem gewebeverträglichen Material durch die Dicke und Breite der Platte sichergestellt werden und liegt üblicherweise im Bereich fachmännischen Handelns.

So sind beispielsweise Knöchenplatten für kleine und weniger belastete Körperpartien, wie beispielsweise Hand- und Fußknochen dünner und häufig weniger breit als Knochenplatten der größeren und stärker belasteten Partien, wie beispielsweise bei Teilen des Unter- und des Oberschenkels.

Die Knochenplatte weist eine Oberseite 11 und eine Unterseite 12 auf, die gewöhnlich parallel zur Plattenebene verlaufen, wobei die Unterseite 12 dem zu fixierenden Knochen benachbart ist. Die Lochausbildungen 2 sind entlang der Längsachse des länglichen Plattenkörpers 1 aufgereiht und bestehen aus zwei stufig ausgebildeten Rundlöchern, 21 und 22, die sich quer zur Plattenebene erstrecken und deren Achsen 21a, 22a die Längsachse der Knochenplatte schneiden. Der kleinste Durchmesser des ersten Rundlochs 21 ist größer als der kleinste Durchmesser des zweiten Rundlochs 22 und der Abstand der beiden Achsen 21a und 22a voneinander ist kleiner als der kleinste Durchmesser des ersten Rundlochs 21.

Durch die Überschneidung der Löcher 21, 22 ergeben sich Kanten 23,24, welche die Bereiche der Rundlöcher gegeneinander abgrenzen und einen Durchlass für den relativ dünnen Schraubschaft einer Knochenschraube freilassen, wenn bei der Schrägstellung einer Knochenschraube diese von einem Rundloch in das andere Rundloch hineinreicht. Die Umfangswinkel der Wandlaibungen der Rundlöcher 21, 22 betragen 250° des größeren Loches und 220° des kleineren Loches. Variationen um 10° kleiner und 20° größer sind möglich.

Infolge der Stufigkeit der Rundlöcher 21,22 kann man eine obere Region 25 und eine untere Region 26 des Rundloches 21 und eine obere Region 27 sowie eine untere Region 28 des Rundlochs 22 unterscheiden.

Die oberen Regionen 25 und 27 sind schüsselförmig ausladend gestaltet, während die unteren Regionen 26 und 28 Mantelflächen mit geraden Mantellinien bilden. Die oberen Regionen 25 und 27 weisen größere Durchmesser gegenüber den unteren Regionen 26 und 28 auf. Die untere Region 26 bildet einen kegelstumpfförmigen Abschnitt, der sich zur Unterseite des Plattenkörpers 1 hin verjüngt. Die untere Region 28 ist zylindrisch ausgebildet, sie kann aber auch kegelstumpfförmig ausgebildet sein.

Während die beiden oberen Regionen 25 und 27 der beiden Rundlöcher 21,22 insgesamt schüsselförmig gestaltet sind, zieht sich, ausgehend von den Lochwandungen, eine radiale Rippe 33 in einer Ebene um die Lochausbildung 2 herum. Rippe 33 ist als umlaufender Steg ausgebildet und weist einen keilförmigen Querschnitt auf, der sich zur Mitte des jeweiligen Rundloches hin verjüngt. In der Draufsicht erscheint die Rippe 33 einer Acht ähnlich zu sein.

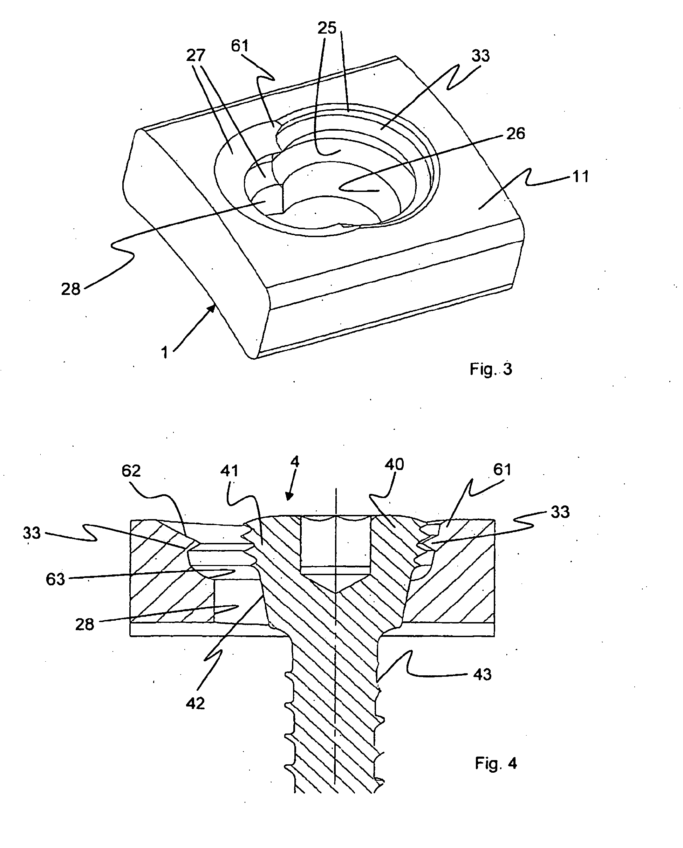

Während die Rippe 33 gleichmäßig herumlaufen kann, wird für den am größeren Rundloch angebrachten Rippenteil ein Grat 61 parallel zur Ebene der Rippe 33 am Außenrand des größeren Rundlochs 21 angeordnet, wodurch sich eine Kante 24 zum zweiten Rundloch 22 hin bildet. Ausgehend von der Längsachse des Plattenkörpers 1 nimmt die Höhe des Grates 61 zum Rand des Plattenkörpers 1 hin zu, wie am besten aus dem Vergleich der Fig. 4 mit Fig. 3 hervorgeht. Zwischen dem Grat 61 und der Rippe 33 wird eine Gangführung gebildet, die den Eingriff des Gewindes 41 der Kopfschraube 40 begünstigt.

Jedes der beiden Rundlöcher 21,22 ist in drei Abschnitte unterteilt: Das größeren Rundloch 21 weist oberhalb der Rippe 33 einen oberen, rundkehlförmigen Abschnitt 31 mit, oder auch ohne Grat 61 auf, und unterhalb der Rippe 33 sind ein mittlerer, rundkehlförmiger Abschnitt 36 und ein unterer, kegelstumpfförmiger oder konischer Abschnitt 26 vorgesehen. Das kleinere Rundloch 22 umfasst einen oberen Abschnitt 35 mit Einführungsschräge 62, einen mittleren Abschnitt 36 mit einer geneigt-abgerundeten Fläche 63 unterhalb der Ebene der Rippe 33, und einen unteren Abschnitt 28, der vorzugsweise zylindrisch ausgebildet ist, aber auch konisch sein kann.

Die Knochenplatte ist zur Zusammenwirkung mit wenigstens zweierlei Arten von Knochenschrauben ausgebildet.

Die eine Art weist einen Knochenschraubenkopf mit teilsphärischer Unterseite auf und kann sich an der geneigt-abgerundeten Fläche 63 mit der Kopfunterseite abstützen. Dabei ist auch Schrägstellung der Schraubenachse gegenüber der Plattenebene möglich, und zwar sowohl in Längsrichtung als auch (im geringen Maße) in Querrichtung zur Knochenplatte. Ermöglicht wird dies durch den Abstand der Kanten 23 voneinander, der dem Anwendungszweck gemäß gewählt wird.

Eine weitere Art von anwendbarer Knochenschraube 4 ist in Fig. 4 dargestellt. Diese Knochenschraube 4 weist einen Schraubkopf 40 mit Inneneingriff und mit Außengewinde 41 am oberen Ende sowie konischer Stützfläche 42 am unteren Ende auf, deren Konusneigung der Konusneigung der unteren Region 26 des größeren Rundloches 21 entspricht.

Die Konusneigung der unteren Region 26 des größeren Rundloches 21 sowie die Konusneigung der konischen Stützfläche 42 am unteren Ende des Schraubkopfs 40 der Knochenschraube 4 weist einen Winkel relativ zur Längs- oder Symmetrieachse jeweils der Knochenschraube oder des Rundlochs 21 im Bereich von 3 bis 30° auf. Bevorzugt liegt dieser Konuswinkel in einem Bereich von 6 bis 20° und am bevorzugtesten in einem Bereich von 8 bis 12°. Eine besonderst bevorzugte Bauform hat sich mit einem Konuswinkel von etwa 10° mit hohen Dauerfestigkeitswerten und guter Lösbarkeit der Verbindung zwischen Knochenschraube und Knochenplatte sehr bewährt.

In einem Winkelbereich von etwa 8 bis 12° werden sehr gute Stabilitätswerte gegenüber dem Verkippen der Knochenschraube relativ zur Knochenplatte bei gleichzeitig nur moderater Selbsthemmung bereitgestellt.

Das Außengewinde 41 kann zylindrisch sein, es wird jedoch konisches Gewinde bevorzugt. An den Schraubkopf 40 schließt sich ein Schraubschaft 43 an, der dazu bestimmt ist, in einem zu fixierenden Knochenelement verankert zu werden. Die umlaufende Rippe 33 erstreckt sich in einer Ebene, vorzugsweise parallel zur Plattenebene, während sich das Gewinde 41 entlang von Schraubflächen erstreckt, die naturgemäß schräg gegenüber der Ebene der radialen Rippe 33 verlaufen, und zwar auch dann, wenn die Knochenschraube 4 parallel oder gleichlaufend mit der Achse 21a des Rundloches 21 in Eingriff gebracht wird. Dabei erfolgt Klemmung zwischen den Gewindegängen 41 und der Rippe 33. In dieser Hinsicht ist der Grat 61 nützlich, da er ein Gegenlager für das Gewinde des Schraubkopfes bietet und dabei eine definierte Klemmung mit definierter selbsthemmender Wirkung bereitstellt.

Im Zusammenhang mit der dargestellten Knochenplatte kann die Knochenschraube 4 zur gegenseitigen Annährung und zum Zusammenpressen von Knochenfragmenten benutzt werden. Hierzu wird die Knochenschraube 4 mit ihrer Achse parallel zur Achse 22a des Rundloches 22 angesetzt. Sobald der untere Rand der konischen Stützfläche 42 die Einführungsschräge 62 des kleineren Rundloches 22 erreicht, erfolgt beim Eindrehen der Schraube eine seitliche Kraft auf den Schraubkopf 40, was zu einer Verschiebung des zu fixierenden Knochenfragments relativ zu der Knochenplatte führt. Wenn demnach ein Knochenfragment mit der Knochenplatte bereits fest verbunden ist, wird dieses Knochenfragment gegen das zu fixierende Knochenfragment geschoben, wie es gewünscht wird.

Es wird darauf hingewiesen, dass wegen des großen Umfangwinkels der Konusfläche der unteren Region 26, die im Bereich von 250 ° bis 290° liegt, bei festsitzendem Schraubkopf 40 eine genügend starke Verbindung zwischen dem Plattenkörper 1 und der Knochenschraube 4 hergestellt wird, da die umlaufende Rippe 33 nach Klemmung des Außengewindes 41 genügend elastische Spannkraft entwickelt, um die konischen Flächen bei 26 und 42 aufeinandergepresst zu halten.

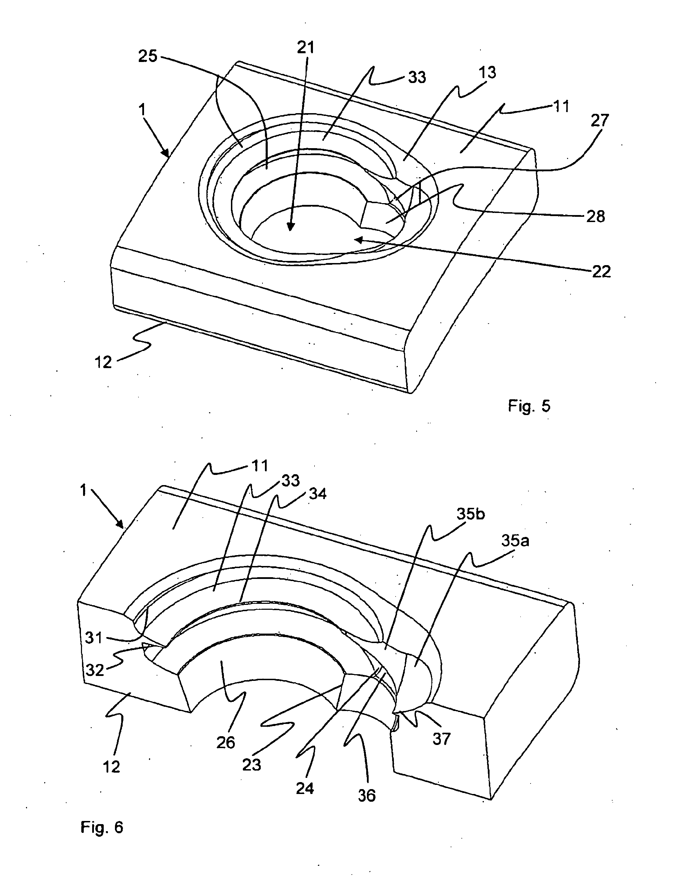

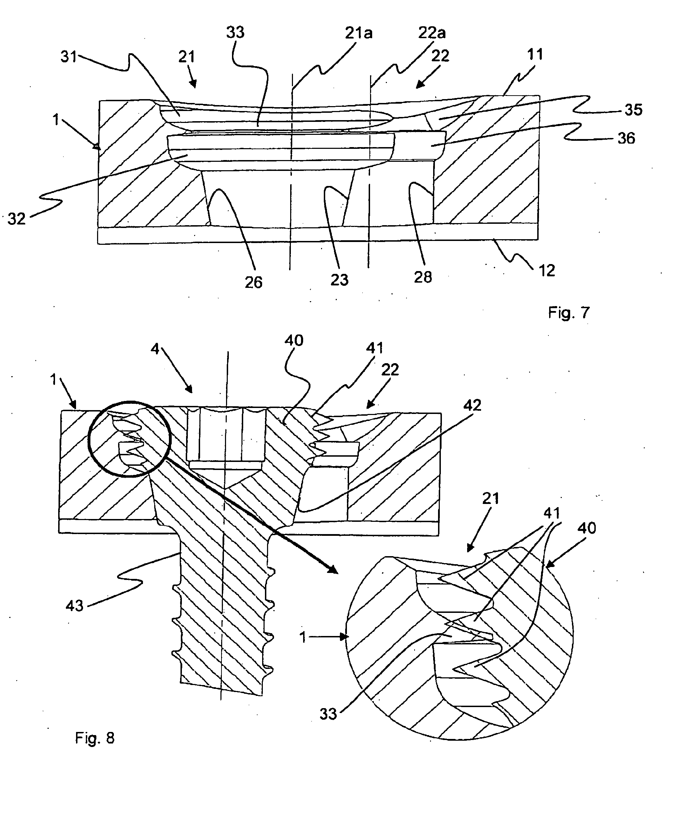

Mit Fig. 5 - 8 ist eine weitere Ausführungsform der Knochenplatte dargestellt, wobei die gleichen Bezugszeichen für sich entsprechende Teile verwendet werden.

Der hauptsächliche Unterschied besteht in der Ausbildung der umlaufenden radialen Rippe 33. Diese ist im Bereich des kleineren Rundlochs 22 teilweise weggeschnitten, um eine geneigt-abgerundete Gleitfläche 35a und eine Übergangsfläche 35b zu schaffen, die zur Führung des Kopfes einer Kochenschraube mit teilsphärischer Unterseite des Kopfes dienlich sind. Es wird eine Restrippe 37 gebildet, die auf volle Größe der Rippe 33 im Bereich des größeren Rundloches 21 ansteigt. Die verbleibende Randkante 34 erstreckt sich um weniger als 180° und erlaubt so die seitliche Einführung von Knochenschrauben vom kleineren Rundloch 22 in das größere Rundloch 21.

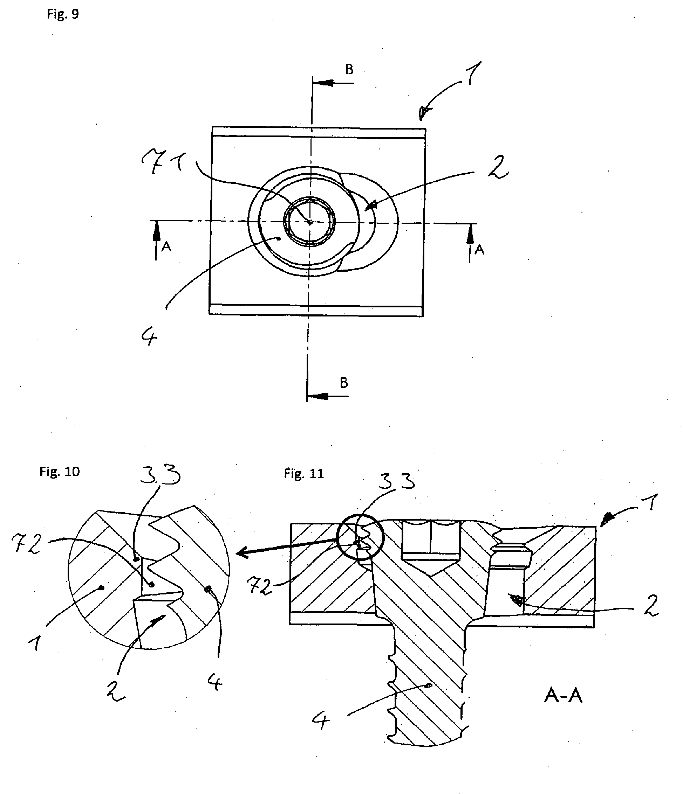

Nachfolgend wird auf Figur 9 Bezug genommen, welche eine weitere Ausführungsform einer Knochenplatte 1 mit einer umlaufenden Rippe 33 zeigt, wobei diese umlaufende Rippe 33 eine verschiedene Höhe in radialer Richtung, also zu deren Symmetrieachse hin, welche beispielsweise mit dem Bezugszeichen 71 versehen in etwa durch den Schnitt der Ebene AA mit der Ebene BB definiert ist, aufweist.

In diese Knochenplatte 1 ist in Figur 9 eine Knochenschraube 4 eingefügt dargestellt.

Die umlaufende Rippe 33 der Knochenplatte 1 weist einen keilförmigen Querschnitt auf, bei welchem die Höhe des keilförmigen Querschnitts in radialer Richtung entlang eines Umlaufs jedoch in dieser weiteren Ausführungsform nicht konstant ist, dies bedeutet nicht für jeden Abschnitt den gleichen Wert aufweist.

In dieser weiteren Ausführungsform ist der keilförmige Querschnitt der umlaufenden Rippe 33 bereichsweise abgeflacht, wie dieses besonders gut der Figur 10 zu entnehmen ist, welche eine Ausschnittvergrößerung eines Teils einer Querschnittdarstellung entlang der Linie AA uns somit parallel zu der Längsachse der in Figur 9 dargestellten weiteren Ausführungsform der Knochenplatte 1 verlaufend zeigt.

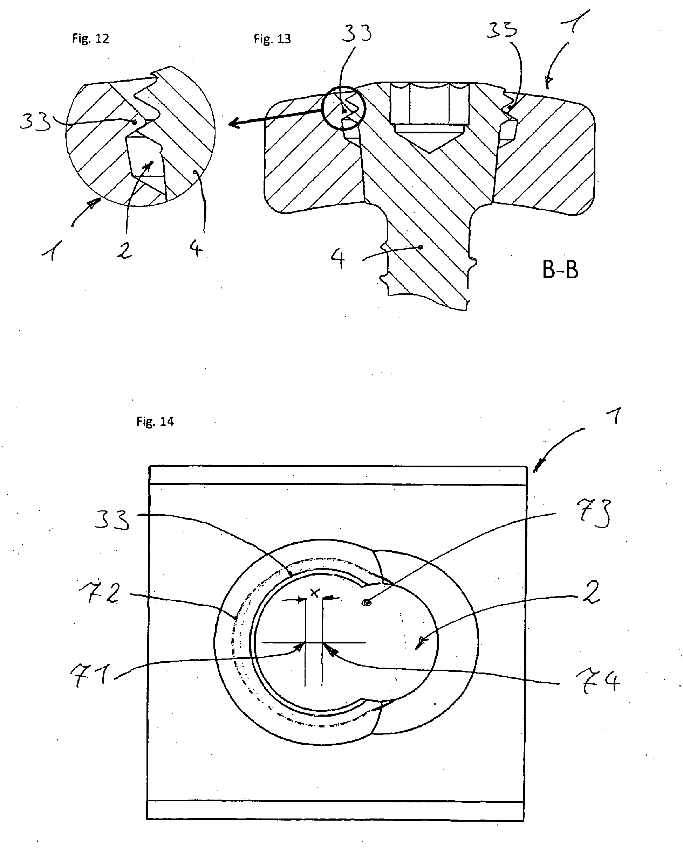

Die Abflachung 72 vermindert die radiale Höhe, so dass es bei dieser Ausgestaltung zu verminderten Klemmkräften durch die Gewindesteigung des Gewindes der Knochenschraube 4 kommt. Im Bereich der Abflachung 72 kann das Gewinde der Knochenschraube frei vor der umlaufenden Rippe 33 angeordnet werden, welches in Bezug auf die Anordnung der Knochenschraube 4 relativ zur Knochenplatte 1 auch gut der Figur 11 zu entnehmen ist. Hierdurch kommt es nur noch zu einem im Wesentlichen zweiseitigen Eingriff der umlaufenden Rippe 33 mit dem Gewinde der Knochenschraube 4, wie es beispielsweise den Figuren 12 und 13 zu entnehmen ist.

Figur 12 zeigt eine Ausschnittvergrößerung eines Teils der Querschnittdarstellung aus Figur 13 entlang der Linie BB und somit quer zu der Längsachse der in Figur 9 dargestellten weiteren Ausführungsform der Knochenplatte verlaufend mit eingefügter Knochenschraube, aus welcher gut zu erkennen ist, dass sowohl die linke als auch die rechte Seite des Gewindes der Knochenschraube mit der Rippe 33 in Eingriff steht. Durch diese Ausgestaltung kann die Steigung des Gewindes der Knochenschraube in deren durch Reibung hemmender Wirkung etwas vermindert werden und können gezielt auch geringere Reib- und selbsthemmende Kräfte bereit gestellt werden als bei einer Rippe 33 mit gleicher Radialer Höhe.

Hierbei liegt es auch im Rahmen der Erfindung, dass die Höhe in radialer Richtung des keilförmigen Querschnitts der umlaufenden Rippe 33 bereichsweise im Wesentlichen den Wert Null annimmt.

In Figur 14 ist eine nochmals weitere Ausführungsform einer Knochenplatte 1 mit einer umlaufenden Rippe 33 mit verschiedener Höhe in radialer Richtung gezeigt, bei welcher die Symmetrieachse 71 der umlaufenden Rippe relativ zur Symmetrieachse des unter der Rippe liegenden konischen Abschnitts 26 des größeren Rundlochs versetzt angeordnet ist und bei welcher entsprechende Radien 72 und 73 um die jeweilige Symmetrieachse strichpunktartig eingezeichnet und durch jeweils einen Kreis angedeutet sind, um deren relativen Versatz einfacher erkennbar zu machen.

Die umlaufende Rippe 33 kann auch die gleiche radiale Höhe über den gesamten Umfang aufweisen und nur durch den Versatz mehr oder weniger in Eingriff mit dem Gewinde des Kopfes der Knochenschraube 4 geraten.

Hierbei erstreckt sich der durch den Radius 72 gebildete Kreis symmetrisch zur Symmetrieachse 71 der umlaufenden radialen Rippe 33 und der durch den Radius 73 gebildete Kreis symmetrisch zur Symmetrieachse 74 des konischen Abschnitts 26 des größeren Rundlochs.

Der in Figur 14 mit "x" bezeichnete Versatz zwischen den Symmetrieachsen 73 und 74 definiert die Änderung der radialen Höhe der Rippe 33.

Dieser Versatz ist nicht auf die in Figur 14 dargestellte Richtung beschränkt, sondern kann auch jeder beliebigen anderen Richtung angeordnet sein.

Ist dieser Versatz in einer Richtung gleich oder größer als die radiale Höhe der Rippe 33, kommt es hierdurch zu Bereichen 75, in welchen die radiale Höhe des keilförmigen Querschnitts der umlaufenden Rippe (33) im Wesentlichen den Wert Null annimmt.

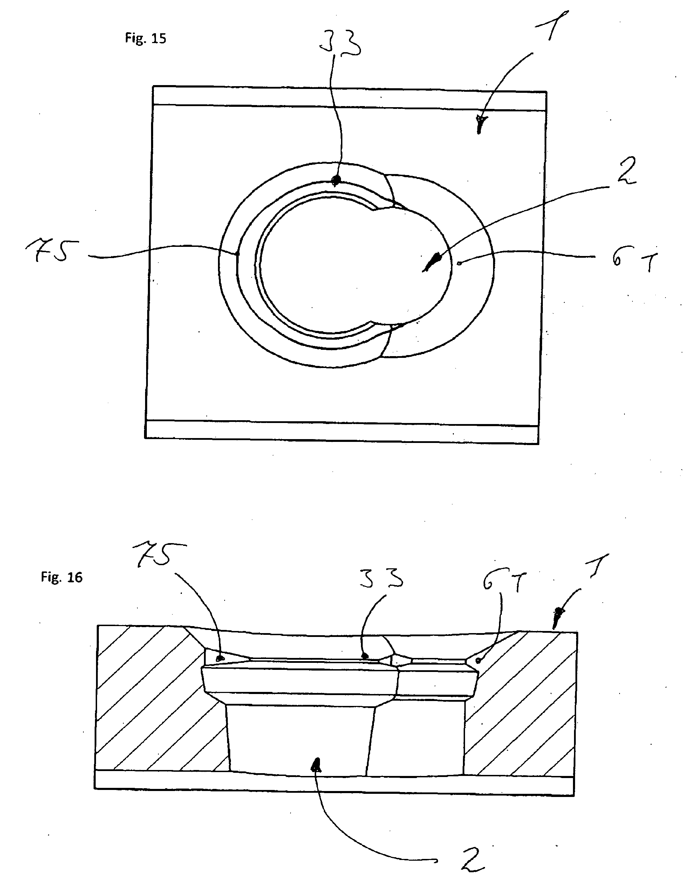

Diese Bereiche sind besonders gut den Figuren 15 bis 17 zu entnehmen.

Figur 15 zeigt dabei die in Figur 14 dargestellte nochmals weitere Ausführungsform der Knochenplatte 1 jedoch ohne die entsprechenden Radien 72, 73 um die jeweilige Symmetrieachse 71, 74, um deren tatsächliche Form einfacher erkennbar zu machen.

Figur 16 zeigt eine Querschnittdarstellung der in Figur 14 und 15 dargestellten nochmals weiteren Ausführungsform parallel zur Längsachse der in Figur 14 und 15 dargestellten nochmals weiteren Ausführungsform der Knochenplatte 1 verlaufend, jedoch ohne eingefügte Knochenschraube 4, um den Verlauf der Rippe 33 besser erkennen zu lassen.

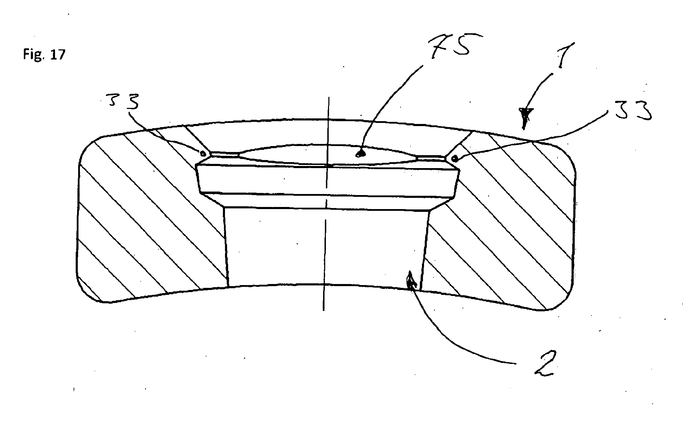

Figur 17 eine Querschnittdarstellung der in Figur 14 und 15 dargestellten nochmals weiteren Ausführungsform quer zur Längsachse der in Figur 14 und 15 dargestellten nochmals weiteren Ausführungsform der Knochenplatte 1 verlaufend ebenfalls ohne eingefügte Knochenschraube 4, um auch in diesem Fall den Verlauf der Rippe 33 besser erkennen zu können.

Wie bei der in den Figuren 9 bis 13 dargestellte Ausführungsform wird in Abhängigkeit vom Versatz x ein mit dem Versatz x ein definiert vorgebbarer abnehmender Eingriff des Gewindes der Knochenschraube 4 in die Rippe 33 bereitgestellt und können auch in diesem Fall Reibung des Gewindes der Knochenschraube 4 mit der Rippe 33 definiert vermindert werden gegenüber einer Rippe mit konstanter radialer Höhe.

Figur 17 zeigt eine Anordnung, bei welcher es im Wesentlichen ähnlich wie in Figur 13 dargestellt nur noch zu einem zweiseitigen Eingriff der Rippe 33 in das Gewinde der Knochenschraube 4 kommt.

Die umlaufende Rippe 33 kann die Wirkung einer Anphasung aufweisen, welch Kerb- und Bruchtendenzen bei höheren Belastungen, wie Biegebelastungen der Knochenplatte entgegen wirkt.

Hierdurch sind Ausführungsformen mit umlaufender Rippe, bei welchen die radiale Höhe der Rippe 33 konstant bleibt von sehr großem Vorteil für die Festigkeit und Biegbelastbarkeit der Knochenplatte 1.

Mit der Erfindung wird ein Fixationssystem für Knochen mit Knochenplatte und Knochenschrauben geschaffen, bei dem Knochenschrauben mit Rundkopf im unterschiedlichen Schrägwinkel zur Knochenplatte gesetzt werden können. Ferner ermöglicht das Fixationssystem bei der Abwendung von Knochenschrauben mit Kegelkopf die Relativverschiebung zwischen zu fixierendem Knochenfragmente und Knochenplatte, was es dem Operateur ermöglicht, Knochenfragmente bei deren Fixierung gegeneinander zu verschieben.

## Patentansprüche

1. Knochenplatte zur Zusammenwirkung mit Knochenschrauben (4), die jeweils einen Kopf (40) und einen Schraubschaft (43) aufweisen, um Teile eines gebrochenen oder geschädigten Knochens zu fixieren, umfassend:
einen vorzugsweise länglichen Plattenkörper (1) aus insbesondere gewebeverträglichem, vorzugsweise steifem Material, der eine Plattenebene und eine Längsachse bestimmt,
Lochausbildungen (2) quer zur Plattenebene, die aus einem ersten Rundloch (21) und einem zweiten Rundloch (22) bestehen, welche sich unter Bildung von Kanten (23,24) schneiden, wobei das erste Rundloch (21) größer als das zweite Rundloch (22) ist, und wobei obere Regionen (25,27) des ersten und zweiten Rundlochs (21,22) einen größeren Durchmesser als untere Regionen (26,28) des ersten und zweiten Rundlochs (21,22) aufweisen, und wobei die untere Region (26) des ersten Rundlochs (21) kegelstumpfförmig ausgebildet ist, **gekennzeichnet durch** genau eine umlaufende radiale Rippe (33) in beiden Rundlöchern, die von beiden Rundlochwandungen ausgeht, um beide Rundlöcher (21,22) herumführt und sich in einer Ebene zur Rundlochmitte hin erstreckt.

2. Knochenplatte nach Anspruch 1,
wobei zur Oberseite (11) der Knochenplatte hin im ersten Rundloch (21), parallel zur radialen Rippe (33) ein Grat (61) verläuft, der mit einer Kante (24) zum zweiten Rundloch (22) hin endet.

3. Knochenplatte nach Ansprüchen 1 oder 2,
wobei die umflaufende, radiale Rippe (33) im Bereich des zweiten Rundlochs (22) reduziert ist, um eine geneigt-abgerundete Gleitfläche (35a) und eine Übergangsfläche (35b) zu bilden.

4. Knochenplatte nach Ansprüchen 1 bis 3,
wobei die umlaufende Rippe (33) im zweiten Rundloch (22) zur Oberseite (11) der Knochenplatte hin mit Einführungsschräge (62) verläuft.

5. Knochenplatte nach einem der vorstehenden Ansprüche,
wobei die obere Region (25) des ersten, größeren Rundlochs (21) durch die umlaufende Rippe (33) in einen oberen, rundkehlförmigen Abschnitt (31) oberhalb der Rippe (33) und in einen mittleren, rundkehlförmigen Abschnitt (36) unterhalb der Rippe (33) unterteilt ist und die untere Region (26) des größeren Rundlochs (21) als ein unterer, kegelstumpfförmiger Abschnitt ausgebildet ist, der sich zur Unterseite (12) des Plattenkörpers (1) hin verjüngt und dessen größter Durchmesser kleiner als der Durchmesser des oberen oder mittleren Abschnitts (31,32) ist.

6. Knochenplatte nach Anspruch 5,
wobei die obere Region (27) des zweiten, kleineren Rundloches (22) einen oberen Abschnitt (35) mit Einführungsschräge (62) aufweist und einen mittleren Abschnitt (36) mit einer geneigt-abgerundeter Fläche (63) unterhalb der Ebene der umlaufenden Rippe (33) besitzt.

7. Knochenplatte nach einem der Ansprüche 1 bis 6,
wobei die umlaufende Rippe (33) einen keilförmigen Querschnitt aufweist, insbesondere bei welchem die Höhe in radialer Richtung des keilförmigen Querschnitts entlang eines Umlaufs nicht konstant ist.

8. Knochenplatte nach Anspruch 7 mit einer umlaufenden Rippe mit verschiedener Höhe in radialer Richtung, bei welcher die Symmetrieachse der umlaufenden Rippe relativ zur Symmetrieachse des unter der Rippe liegenden konischen Abschnitts des größeren Rundlochs versetzt angeordnet ist.

9. Knochenplatte nach Anspruch 7, wobei der keilförmige Querschnitt der umlaufenden Rippe (33) bereichsweise abgeflacht ist, insbesondere wobei die Höhe in radialer Richtung des keilförmigen Querschnitts der umlaufenden Rippe (33) bereichsweise im Wesentlichen den Wert Null annimmt.

10. Knochenplatte nach einem der Ansprüche 1 bis 6, wobei die umlaufende Rippe (33) einen keilförmigen Querschnitt aufweist, ohne dass dieser eine radiale Höhe von im Wesentlichen Null annimmt.

## Claims

1. Bone plate for cooperating with bone screws (4), each comprising a head (40) and a screw shaft (43), for fixing parts of a fractured or damaged bone, comprising:
a preferably elongate plate body (1) of, in particular, tissue-compatible, preferably rigid material, the plate body defining a plate plane and a longitudinal axis,
hole formations (2) transverse to the plate plane, which consist of a first round hole (21) and a second round hole (22) which intersect to form edges (23, 24), the first round hole (21) being larger than the second round hole (22), and
wherein upper regions (25, 27) of the first and second round holes (21, 22) have a larger diameter than lower regions (26, 28) of the first and second round holes (21, 22),
and wherein the upper region (26) of the first round hole (21) is in the shape of a truncated cone, **characterised by**
precisely one circumferential radial rib (33) in both round holes, which issues from both round hole walls, passes around both round holes (21, 22) and extends in a plane towards the round hole centre.

2. Bone plate as claimed in claim 1, wherein a ridge (61) extends towards the upper side (11) of the bone plate in the first round hole (21), in parallel with the radial rib (33), this ridge terminating with an edge (24) towards the second round hole (22).

3. Bone plate as claimed in claim 1 or 2, wherein the circumferential, radial rib (33) is reduced in the region of the second round hole (22) so as to form an inclined-rounded sliding surface (35a) and a transition surface (35b).

4. Bone plate as claimed in claims 1 to 3, wherein the circumferential rib (33) extends with an introductory slope (62) in the second round hole (22) towards the upper side (11) of the bone plate.

5. Bone plate as claimed in any one of the preceding claims, wherein the upper region (25) of the first, larger round hole (21) is divided by the circumferential rib (33) into an upper, round neck-shaped portion (31) above the rib (33) and a central, round neck-shaped portion (36) below the rib (33), and the lower region (26) of the larger round hole (21) is formed as a lower, truncated cone-shaped portion which tapers towards the lower side (12) of the plate body (1) and the largest diameter of which is smaller than the diameter of the upper or central portion (31, 32).

6. Bone plate as claimed in claim 5, wherein the upper region (27) of the second, smaller round hole (22) comprises an upper portion (35) having an introductory slope (62), and has a central portion (36) having an inclined-rounded surface (63) below the plane of the circumferential rib (33).

7. Bone plate as claimed in any one of claims 1 to 6, wherein the circumferential rib (33) has a wedge-shaped cross-section, in particular in which the height in the radial direction of the wedge-shaped cross-section along a circumference is not constant.

8. Bone plate as claimed in claim 7, including a circumferential rib of a different height in the radial direction, wherein the axis of symmetry of the circumferential rib is arranged offset relative to the axis of symmetry of the conical portion of the larger round hole below the rib.

9. Bone plate as claimed in claim 7, wherein the wedge-shaped cross-section of the circumferential rib (33) is flattened in regions, in particular wherein the height in the radial direction of the wedge-shaped cross-section of the circumferential rib (33) becomes substantially zero in regions.

10. Bone plate as claimed in any one of claims 1 to 6, wherein the circumferential rib (33) has a wedge-shaped cross-section, without this assuming a radial height of substantially zero.

## Revendications

1. Plaque d'ostéosynthèse destinée à coopérer avec des vis d'ostéosynthèse (4), qui comportent chacune une tête (40) et une tige (43), afin d'immobiliser des parties d'un os fracturé ou endommagé, comportant :
un corps (1), de préférence allongé, réalisé dans un matériau en particulier biocompatible, de préférence rigide, qui détermine un plan de la plaque et un axe longitudinal,
des formations de trous (2) transversalement au plan de la plaque, qui sont constituées d'un premier trou rond (21) et d'un deuxième trou rond (22), qui se coupent moyennant la formation de bords (23, 24), le premier trou rond (21) étant plus grand que le deuxième trou rond (22), et des régions supérieures (25, 27) du premier et du deuxième trou rond (21, 22) ayant un diamètre supérieur à celui des régions inférieures (26, 28) du premier et du deuxième trou rond (21, 22),
et, la région inférieure (26) du premier trou rond (21) étant réalisée en forme de cône tronqué, **caractérisée par**
exactement une nervure (33) radiale périphérique dans les deux trous ronds, qui part des parois des deux trous ronds, contourne les deux trous ronds (21, 22) et s'étend dans un plan vers le milieu du trou rond.

2. Plaque d'ostéosynthèse selon la revendication 1, dans laquelle une arête (61) s'étend dans le premier trou rond (21) vers la face supérieure (11) de la plaque d'ostéosynthèse parallèlement à la nervure (33) radiale et se termine avec un bord (24) vers le deuxième trou rond (22).

3. Plaque d'ostéosynthèse selon la revendication 1 ou 2, dans laquelle la nervure (33) radiale périphérique est réduite dans la zone du deuxième trou rond (22) pour former une surface de glissement (35a) inclinée arrondie et une surface de transition (35b).

4. Plaque d'ostéosynthèse selon l'une quelconque des revendications 1 à 3, dans laquelle la nervure (33) périphérique s'étend dans le deuxième trou rond (22) avec une rampe d'introduction (62) vers la face supérieure (11) de la plaque d'ostéosynthèse.

5. Plaque d'ostéosynthèse selon l'une quelconque des revendications précédentes, dans laquelle la région supérieure (25) du premier trou rond (21) plus grand est divisée par une nervure (33) périphérique en une partie supérieure (31) en forme de gorge ronde au-dessus de la nervure (33) et en une partie centrale (36) en forme de gorge ronde au-dessous de la nervure (33), et la région inférieure (26) du trou rond (21) plus grand est réalisée sous la forme d'une partie inférieure en forme de cône tronqué, laquelle se rétrécit vers la face inférieure (12) du corps (1) de la plaque d'ostéosynthèse et dont le plus grand diamètre est inférieur au diamètre des parties supérieure ou centrale (31, 32).

6. Plaque d'ostéosynthèse selon la revendication 5, dans laquelle la région supérieure (27) du deuxième trou rond (22) plus petit comporte une partie supérieure (35) avec une rampe d'introduction (62) et possède une partie centrale (36) avec une surface (63) inclinée arrondie au-dessous du plan de la nervure (33) périphérique.

7. Plaque d'ostéosynthèse selon l'une quelconque des revendications 1 à 6, dans laquelle la nervure (33) périphérique a une section transversale conique, en particulier dans laquelle la hauteur dans le sens radial de la section transversale conique n'est pas constante le long d'un pourtour.

8. Plaque d'ostéosynthèse selon la revendication 7, comportant une nervure périphérique avec différentes hauteurs dans le sens radial, dans laquelle l'axe de symétrie de la nervure périphérique est décalé par rapport à l'axe de symétrie de la partie conique, située au-dessous de ladite nervure, du trou rond plus grand.

9. Plaque d'ostéosynthèse selon la revendication 7, dans laquelle la section transversale conique de la nervure (33) périphérique est aplatie par zones, en particulier dans laquelle la hauteur dans le sens radial de la section transversale conique de la nervure (33) périphérique prend par zones sensiblement la valeur zéro.

10. Plaque d'ostéosynthèse selon l'une quelconque des revendications 1 à 6, dans laquelle la nervure (33) périphérique a une section transversale conique, sans que celle-ci ait une hauteur radiale acceptant sensiblement la valeur zéro.
